# NEUE EUROPÄISCHE PATENTSCHRIFT

(11) **EP 0 180 995 B2**
(45) Veröffentlichungstag und Bekanntmachung des Hinweises auf die Entscheidung über den Einspruch: **10.08.1994**
(45) Hinweis auf die Patenterteilung: 23.01.1991
(21) Anmeldenummer: 85114206.7
(22) Anmeldetag: 07.11.1985
(51) Int. Cl.: C07C 21/06, C07C 17/34, C07C 17/38

(54) **Verfahren zur Aufarbeitung des Pyrolyse-Produkts aus der 1.2-Dichlorethan-Spaltung unter Wärmerückgewinnung**
Process for working up the pyrolysis products of the splitting of 1,2-dichloroethane with heat recovery
Procédé pour le retraitement du produit de pyrolyse de la scission de 1,2-dichloroéthane avec récupération de chaleur

(30) Priorität: 09.11.1984 DE 3441080
(43) Veröffentlichungstag der Anmeldung: 14.05.1986
(73) Patentinhaber: WACKER-CHEMIE GMBH, D-81737 München (DE)
(72) Erfinder: Dummer, Gerhard, Dipl.-Ing. (TU), D-8269 Burgkirchen (DE); Haselwarter, Klaus, Dipl.-Ing. (FH), D-8261 Emmerting (DE); Klaus, Hermann, Dipl.-Ing. (FH), D-8261 Marktl (DE); Schmidhammer, Ludwig, Dr. Dipl.-Chem., D-8261 Haiming (DE); Strasser, Rudolf, Dr. Dipl.-Chem., D-8263 Burghausen (DE)

(56) Entgegenhaltungen:
- EP-A- 0 014 920
- EP-A- 0 073 941
- DE-A- 3 147 310
- Kirk-Othmer, "Encyclopedia of chemical Technology", 3rd Ed., Vol. 9, 1980, Seite 30
- Chemical Engineering Science, Vol. 38, N:o 5, 1983, Seiten 745-763
- Institution of chemical Engineers, Rugby, 1982, Seiten 36-46, 75-81
- Ullmans Encyclopädie der technischen Chemie, Band 2, 1972, Seite 411 ff, Abb. 8

## Beschreibung

Die Erfindung betrifft ein Verfahren zurAufarbeitung des Reaktionsprodukts, das bei der thermischen Spaltung von 1.2-Dichlorethan zu Vinylchlorid und Chlorwasserstoff anfällt, unter mehrstufigem Abkühlen und destillativem Auftrennen des Reaktionsprodukts sowie Rückführen nicht umgesetzten 1.2-Dichlorethans.

Bekanntlich wird Vinylchlorid technisch durch thermische Spaltung von 1.2-Dichlorethan und anschließende destillative Auftrennung des Reaktionsprodukts in die Hauptkomponenten Chlorwasserstoff, Vinylchlorid und nicht umgesetztes 1.2-Dichlorethan gewonnen. Für diesen Prozeß müssen etwa 0,3-0,4 Giga-Joule pro 100 kg produzierten Vinylchlorids aufgewendet werden. Der größte Teil dieser Energie fällt primär als Wärmeinhalt des den Spaltofen verlassenden Pyrolyse-Produkts an, der wiederum zum größten Teil schnellstmöglich abgeführt werden muß, um Nebenproduktbildung und Korrosionsschäden an den Anlagen zu unterdrücken.

Nun sind bereits Verfahren bekanntgeworden, wenigstens einen Teil der im Pyrolyse-Produkt enthaltenden Energie wieder zurückzugewinnen.

So wird gemäß EP-OS 14 920 das den Spaltofen verlassende Pyrolyse-Produkt durch indirekte Kühlung abgeschreckt und die an das Kühlmedium abgegebene Energie innerhalb des Prozesses weitergenutzt. Beispielsweise wird als Wärmetauschmedium das Sumpfprodukt einer Destillationskolonne verwendet und in aufgeheiztem Zustand wieder zur Kolonnezurückgeführt.

Nachteiligerweise müssen bei dieserArbeitsweise beträchtliche Druckverluste im Bereich des für die Abschreckung des Pyrolyse-Produkts vorgesehenen Wärmetauschers in Kauf genommen werden. Nun wird üblicherweise der Systemdruck der gesamten Anlage am Kopf der HCI-Kolonne gehalten. Druckverluste, die zwischen Pyrolyse-Einheit und HCI-Kolonne entstehen, müssen deshalb durch Vorgabe eines entsprechend höheren Drucks in der Pyrolyse-Einheit ausgeglichen werden. Es muß mithin in der Pyrolyse-Einheit auf einem beträchtlich höheren Druck- und damit auch auf einem höheren Temperaturniveau gearbeitet werden, als dies an sich zum Betrieb der nachfolgenden Destillationseinheiten erforderlich wäre. Die Folgen sind erhöhte Nebenproduktbildung, insbesondere vermehrte Koksbildung, erhöhter Energiebedarf in der Pyrolyse-Einheit, geringere Standzeiten des Spaltofensystems und andere mehr.

Aufgabe der Erfindung war es, ein Verfahren zu entwickeln, bei dem die zur Pyrolyse von 1.2-Dichlorethan aufgewendete Energie mindestens teilweise zurückgewonnen wird. Insbesondere war es Aufgabe der Erfindung, die zur Aufarbeitung anfallenden Produktströme so zu führen und den Wärmetausch so zu gestalten, daß höhere Druckverluste vermieden werden.

Gegenstand der Erfindung ist ein Verfahren zur Aufarbeitung des Reaktionsprodukts, das bei der thermischen Spaltung von 1.2-Dichlorethan zu Vinylchlorid und Chlorwasserstoff anfällt, unter mehrstufigem Abkühlen und destillativem Auftrennen des Reaktionsprodukts sowie Rückführen nicht umgesetzten 1.2-Dichlorethans zum Spaltofen, wobei
a) das den Spaltofen verlassende Reaktionsprodukt durch direkte Kühlung abgeschreckt und einer Quenchkolonne aufgegeben wird wobeider innerhalb der Quenchkolonne aufsteigende gasförmige Produktstrom im Gegenstrom vom Kondensat, das bei der weiteren Aufarbeitungsprozedur der Dichlorethan-Spaltprodukte anfällt und als Rücklauf dem oberen Teil der Quenchkolonne aufgegeben wird, gewaschen und gekühlt wird, dadurch gekennzeichnet, daß
b) das Produkt gemäß a) bei einem Druck von 9-12 bar (abs.) und einer Temperatur von 120-140°C als Brüden der Quenchkolonne gewonnen wird und
c) die Brüden der Quenchkolonne durch indirekte Kühlung bis mindestens auf ihren Taupunkt gebracht werden, wobei in parallel betriebenen Wärmetauschern
   _{C1}) 20 bis 40 Vol% der Brüden mit 1.2-Dichlorethan, das in den Spaltofen geführt wird und c₂) 10 bis 20 Vol% der Brüden mit Luft, die als Verbrennungsluft zur Spaltofenbefeuerung eingesetzt wird, und
   c₃) 30 bis 50 Vol% der Brüden mit Sumpfprodukt der für die destillative Abtrennung von Chlorwasserstoff vorgesehenen Kolonne und gegebenenfalls
   c₄) ein Restanteil der Brüden mit flüssigem Chlorwasserstoff
als Wärmetauschmedien eingesetzt werden.

Erfindungsgemäß wird die Spaltung von 1.2-Dichlorethan bevorzugt im Temperaturbereich von 480-540°C unter Drücken von 10-25 bar abs. durchgeführt. Der Druckbereich von 10-14 bar abs. ist bevorzugt. In diesem Druckbereich kann Dichlorethan in der Pyrolyse-Einheit schonend durch Wärmetausch mit technisch üblicherweise zur Verfügung stehendem hochgespanntem Wasserdampf (von ca. 16-23 bar abs.) vorverdampft werden.

Das den Spaltofen verlassende Reaktionsgemisch, das als Hauptbestandteile Chlorwasserstoff, Vinylchlorid und nicht umgesetztes 1.2-Dichlorethan enthält, wird nun auf eine Temperatur von 150-250°C durch direkte Kühlung abgeschreckt, bevor es der nachfolgenden Quenchkolonne aufgegeben wird. Unter direkter Kühlung wird im Rahmen der Erfindung eine Kühltechnik verstanden, bei der das Wärmetauschmedium durch Vermischen mit dem abzukühlenden Medium in direkten Kontakt gebracht wird. Erfindungsgemäß erfolgt die direkte Kühlung durch Einspritzen von Sumpfprodukt, im wesentlichen Dichlorethan, aus der Quenchkolonne. Das Sumpfprodukt weist etwa eine Temperatur von 145-165°C auf. Die Menge an einzuspritzendem Sumpfprodukt wird nach Maßgabe des zu erzielenden Abkühleffekts bemessen.

Bei dem beschriebenen direkten Kühlverfahren verdampft ein Großteil des Sumpfproduktes der Quenchkolonne, so daß ein Gas-Flüssigkeits-Gemisch aus Pyrolyse-Produkt und Kolonnen-Sumpfprodukt erhalten wird, das mit einer Temperatur von 150-250°C dem unteren Teil der Quenchkolonne aufgegeben wird. Der innerhalb der Kolonne aufsteigende gasförmige Produktstrom wird nun im Gegenstrom von Kondensat, das bei der weiteren Aufarbeitungsprozedur der Dichlorethan-Spaltprodukte anfällt und als Rücklauf dem oberen Teil der Quenchkolonne aufgegeben wurde, gewaschen und dadurch von Kokspartikeln, die als Nebenprodukt beim Pyrolyse Prozeß gebildet wurden, befreit. Hierbei wird gleichzeitig ein weiterer Kühleffekt erzielt, so daß schließlich am Kopf der Quenchkolonne bei einem Druck von 9-12 bar abs. Kolonnenbrüden mit einer Temperatur von 120-140°C gewonnen werden.

Die Brüden werden nun durch indirekte Kühlung bis mindestens auf ihren Taupunkt abgekühlt und schließlich einer Kolonne zur Abtrennung von Chlorwasserstoff zugeführt. Zweckmäßigerweise wird dabei stets ein Teil an Kondensat, im wesentlichen 1.2-Dichlorethan, als Rücklauf zur Quenchkolonne zurückgeführt, um den unmittelbar aus der Pyrolyse-Einheit kommenden Produktstrom in der Quenchkolonne zu waschen und von Kokspartikeln zu befreien. Unter indirekter Kühlung wird eine Verfahrensweise verstanden, bei der Kühlmedium und abzukühlendes Medium lediglich indirekt über Wärmetauschflächen in Kontakt gebracht werden, ohne daß Vermischung stattfindet.

Erfindungsgemäß werden die Kolonnenbrüden durch mindestens drei Wärmetauscher geleitet, die mit verschiedenen Wärmetauschmedien beaufschlagt sein können. Als Wärmetauschmedien kommen das Sumpfprodukt der für die Abtrennung von Chlorwasserstoff vorgesehenen Kolonne; flüssiges 1.2-Dichlorethan, das in aufgeheiztem Zustand der Pyrolyse-Einheit zugeführt wird; Luft, die als Verbrennungsluft zur Spaltofenbefeuerung benötigtwird; und flüssiger Chlorwasserstoff in Betracht.

Zumeist wird ein weiterer Wärmetauscher vorgesehen, der zum Ausgleich der Gesamtwärmebilanz dient, um innerhalb der erfindungsgemäßen Maßnahmen nicht genutzte Wärme abzuführen. Dieser Wärmetauscher kann beispielsweise mit Wasser als Wärmetauschmedium beaufschlagt sein.

Die Wärmerückgewinnung aus den durch Pyrolyse von 1.2-Dichlorethan gewonnenen heißen Spaltgasen erfolgt mithin durch indirekten Wärmetausch mit solchen Medien, die innerhalb des Prozesses verwendet werden. In diesen Rahmen fällt auch der Wärmetausch mit flüssigem Chlorwasserstoff, der dabei in gasförmigem Zustand übergent, da ein Teil der im Prozeß anfallenden großen Mengen an Chlorwasserstoff aus an sich bekannten verfahrenstechnischen Gründen in flüssigem Zustand zwischengelagertwerden muß, um die bei der Weiterverwendung von Chlorwasserstoff anfallenden Produktströme zu glätten.

Da bei der beschriebenen Verfahrensweise keine nennenswerten Druckverluste auftreten, können die erfindungsgemäßen Maßnahmen ohne weiteres innerhalb bestehender Anlagen durch Zwischenschalten entsprechender Wärmetauscher durchgeführt werden.

Die Auslegung der Wärmetauscher hinsichtlich Konstruktionsart und Größe liegt im Können des Fachmannes. Der Brüdenstrom wird geteilt, wobei 30-50 Vol.-% der Brüden durch Wärmetausch mit dem Sumpfprodukt der HCI-Kolonne, 20-40 Vol.-% durch Wärmetausch mit in die Pyrolyse-Einheit einzuspeisendem 1.2-Dichlorethan und 10-20 Vol.-% durch Wärmetausch mit Luft, die als Verbrennungsluft für die Spaltofenbefeuerung benötigt wird, auf mindestens ihren Taupunkt abgekühlt werden.

Nach dem erfindungsgemäßen Verfahren gelingt es, einen Großteil der bei der Pyrolyse von 1.2-Dichlorethan aufzubringenden Energie wiederzugewinnen, um sie innerhalb des Prozesses weiter zu nutzen. Beispielsweise kann die zum Betrieb der Chlorwasserstoffkolonne benötigte Energie vollständig durch entsprechenden Wärmetausch mit den Spaltgasen gewonnen werden.

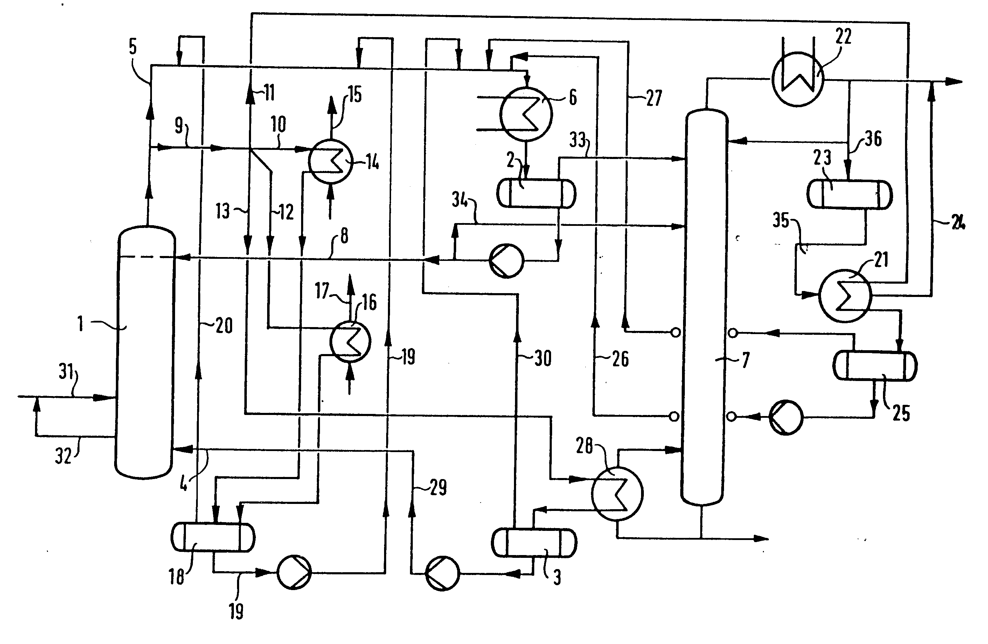

Die Abbildung zeigt ein Fließschema einer besonders bevorzugten Ausführungsform des Verfahrens. Im folgenden Beispiel wird unter Bezug auf die Abbildung die Erfindung näher erläutert:

### Beispiel:

75 t/h 1.2-Dichlorethan wurden bei 12,5 bar abs. und einer Temperatur von 512°C (gemessen am Ofenaustritt) bei einer Spaltrate von 60% zu Vinylchlorid und Chlorwasserstoff pyrolysiert. Das den Pyrolyse-Ofen über Leitung 31 verlassende Reaktionsgemisch wurde durch Einspritzen von 120 t/h SumpfproduktausderQuenchkolonne 1 über Leitung 32 auf 160°C abgeschreckt. Die derart durch direkte Kühlung erhaltene Gas-Flüssigkeitsmischung wurde in den unteren Teil derQuenchkolonne 1 geleitet, die unter einem Druck von 11 bar abs. stand. Die innerhalb der Kolonne aufsteigende Mischung wurde im Gegenstrom mit 40 t/h Rücklauf aus dem Kondensatsammler 2, der über Leitung 8 dem oberen Teil der Kolonne aufgegeben wurde, gewaschen und dadurch von Kokspartikeln befreit. Dabei verdampfte der Rücklauf zusammen mit 30,4 t/h Quenchbrüdenkondensat aus dem Kondensatsammler 3, der bei 4 mit einer Temperatur von 100°C in den Sumpf gepumpt wurde. Die dadurch am Kopf der Quenchkolonne erhaltenen Brüden wiesen noch eine Temperatur von 124°C auf.

15 Vol.-% der Quenchkolonnenbrüden wurden über Leitung 5 zum wassergekühlten Kondensator 6 geleitet, indem dieser Quenchbrüden-Teilstrom auf 45°C abgekühlt wurde. Dabei trat Partialkondensation ein. Im Sammler 2 wurden die kondensierten Bestandteile von den gasförmigen Bestandteilen getrennt. Die gasförmigen Bestandteile strömten über Leitung 33 in den oberen Teil der HCI-Kolonne 7.20% der kondensierten Phase wurden über Leitung 8 als Rücklauf der Quenchkolonne 1 aufgegeben, während 80% über Leitung 34 zur HCI-Kolonne 7 geführt wurden.

Über Leitung 9 und 10 strömten 25 Gew.-% der Quenchbrüden zum Wärmetauscher 14, in dem 75 t/h 1.2-Dichlorethan durch Wärmetausch mit den Quenchbrüden von 30 auf 80°C vorgewärmt wurden und über Leitung 15 zur Konvektionszone des Spaltofens geführt wurden. Dabei wurden im Wärmetauscher 14 die Quenchbrüden unter teilweiser Kondensation auf 80°C abgekühlt.

Weitere 15 Gew.-% der Quenchbrüden gelangten über Leitung 9 und 12 zum Wärmetauscher 16, in dem 32.400 kg/h Verbrennungsluft für die Spaltofenbefeuerung unter Abkühlen auf 100°C und Teilkondensation der Quenchbrüden von 20 auf 110°C vorgewärmt wurden. Die vorgewärmte Luft wurde anschließend über Leitung 17 zu den Brennern des Spaltofens geleitet.

Die in den Wärmetauschern 14 und 16 anfallenden Quenchbrüdenkondensate wurden im Sammler 18 von den nicht kondensierten Bestandteilen getrennt. Die kondensierten Bestandteile gelangten über Leitung 19 vor Eintritt in den Kondensator 6 in die Quenchbrüdenleitung 5. Die nicht kondensierten Bestandteile strömten aus dem Sammler 18 über Leitung 20 ebenfalls vor Eintritt in den Kondensator 6 in die Quenchbrüdenleitung 5. Der Produktstrom wurde schließlich im Wärmetauscher 6 auf eine Temperatur von 45°C abgekühlt.

5 Gew.-% der Quenchbrüden gelangten über Leitung 9 und 11 zum Wärmetauscher 21, der mit 2,2 t/h flüssigen Chlorwasserstoff über Leitung 35 aus dem Zwischenlagerbehälter23 beaufschlagtwar. Derflüssige Chlorwasserstoff wurde als partielles Kopfprodukt der HCI-Kolonne 7 erhalten, in dem mit Frigen betriebenen Kondensator 22 kondensiert und über Leitung 36 dem Behälter 23 zugeführt. Der im Wärmetauscher 21 verdampfende Chlorwasserstoff wurde über Leitung 24 mit dem im Kondensator 22 nicht kondensierten Chlorwasserstoffstrom vereint. Die Quenchbrüden wurden im Wärmetauscher 21 auf eine Temperatur von 50°C abgekühlt und im Sammler 25 in die kondensierten und nicht kondensierten Bestandteile getrennt. Die getrennten Bestandteile wurden anschließend separat über die Leitungen 27 bzw. 26 zur Quenchbrüdenleitung 5 geführt und schließlich im Kondensator 6 auf 45°C abgekühlt.

40 Gew.-% der Quenchbrüden wurden über Leitung 9 und 13 dem Wärmetauscher 28 zugeführt, in dem durch Wärmetausch mit den Quenchbrüden der Sumpfinhalt der HCI-Kolonne 7 aufgeheizt wurde. Die Quenchbrüden kühlten sich dabei auf 100°C ab, wobei Partialkondensation eintrat. das Kondensat wurde überden Sammler3 und die Leitung 29 bei4 in den unteren Teil der Quenchkolonne 1 gepumpt, wo es durch Wärmetausch mit der aus Leitung 31 stammenden Spaltgasmischung verdampft wurde. Die nicht kondensierten Bestandteile gelangten über Leitung 30 in die Quenchbrüdenleitung 5 und schließlich in den Kondensator 6.

Als Wärmetauscher wurden bemantelte Rohre benutzt. Im Wärmetauscher 14 strömten die Quenchbrüden um die Rohre, das zu erwärmende 1.2-Dichlorethan befand sich in den Rohren. Beim Wärmetauscher 16 strömten die Quenchbrüden durch die berippten Rohre, während die Luft mantelseitig aufgegeben wurde. Der Wärmetauscher 21 wurde mantelseitig mit den Quenchbrüden beaufschlagt, während der Chlorwasserstoff durch die Rohre geleitet wurde. Beim Wärmetauscher 28 befand sich das Sumpfprodukt der HCI-Kolonne 7 in den Rohren, während die Quenchbrüden den Wärmetauscher mantelseitig durchströmten.

Es konnten 17,6 Giga-Joule/h an Quenchwärme zurückgewonnen werden. Selbst nach einjähriger Betriebspraxis konnte an den 4 Wärmetauschern noch keine Verschmutzung der Wärmetauschflächen festgestellt werden.

Der durch das Quenchbrüdenheizsystem sich einstellende natürliche Druckverlust betrug lediglich 0,3 bar abs., so daß von dieser Seite keine Begrenzung des Gesamtdurchsatzes durch das Spaltsystem gegeben war.

## Patentansprüche

1. Verfahren zur Aufarbeitung des Reaktionsprodukts, das bei der thermischen Spaltung von 1.2-Dichlorethan zu Vinylchlorid und Chlorwasserstoff anfällt, unter mehrstufigem Abkühlen und destillativem Auftrennen des Reaktionsprodukts sowie Rückführen nicht umgesetzten 1.2-Dichlorethans zum Spaltofen, wobei
a) das den Spaltofen verlassende Reaktionsprodukt durch direkte Kühlung abgeschreckt und einer Quenchkolonne aufgegeben wird, wobei der innerhalb der Quenchkolonne aufsteigende gasförmige Produktstrom im Gegenstrom vom Kondensat, das bei der weiteren Aufarbeitungsprozedur der Dichlorethan-Spaltprodukte anfällt und als Rücklauf dem oberen Teil der Quenchkolonne aufgegeben wird, gewaschen und gekühlt wird, dadurch gekennzeichnet, daß
b) das Produkt gemäß a) bei einem Druck von 9-12 bar(abs.) und einer Temperaturvon 120-140°C als Brüden der Quenchkolon ne gewonnen wird und
c) die Brüden der Quenchkolonne durch indirekte Kühlung bis mindestens auf ihren Taupunkt gebracht werden, wobei in parallel betriebenen Wärmetauschern
_{C1}) 20 bis 40 Vol% der Brüden mit 1.2-Dichlorethan, das in den Spaltofen geführt wird und
c₂) 10 bis 20 Vol% der Brüden mit Luft, die als Verbrennungsluft zur Spaltofenbefeuerung eingesetzt wird, und
c₃) 30 bis 50 Vol% der Brüden mit Sumpfprodukt derfür die destillative Abtrennung von Chlorwasserstoff vorgesehenen Kolonne und gegebenenfalls
c₄) ein Restantei I der Brüden mit flüssigem Chlorwasserstoff
als Wärmetauschmedien eingesetzt werden.

## Claims

1. Process for working up the reaction product obtained in the thermal cracking of 1,2-dichloroethane to vinyl chloride and hydrogen chloride, with multi-stage cooling and separation of the reaction product by distillation as well as recycling of unconverted 1,2-dichloroethane to the cracking furnace,
a) the reaction product leaving the cracking furnace being quenched by direct cooling and charged to a quench column, the gaseous product stream rising inside the quench column being washed and cooled in countercurrent by the condensate which arises during the further working-up procedure of the dichloroethane cracking products and is charged as reflux to the top part of the quench column, characterized in that
b) the product according to a) is recovered at a pressure of 9-12 bar (abs.) and at a temperature of 120-140°C as the vapours from the quench column, and
c) the vapours from the quench column are brought down at least to their dew point by indirect cooling, the following being used as heat exchange media in heat exchangers operated in parallel
_{C1}) 20 to 40 % by volume of the vapours with 1,2-dichloroethane, which is fed to the cracking furnace, and
c₂) 10 to 20 % by volume of the vapours with air, which is used as combustion air for firing the cracking furnace, and c₃) 30 to 50 % by volume of the vapours with the bottom product from the column provided for separating the hydrogen chloride off by distillation, and optionally c₄) a residual proportion of the vapours with liquid hydrogen chloride.

## Revendications

1. Procédé pour traiter le produit de réaction formé dans la dissociation thermique du 1,2-dichloréthane en chlorure de vinyle et chlorure d'hydrogène, avec refroidissement en plusieurs étapes et séparation par distillation du produit de réaction, et aussi recyclage au four de dissociation du 1,2-dichloréthane n'ayant pas réagi,
a) le produit quittant le four étant refroidi brusquement par un refroidissement direct et envoyé à une colonne de trempe, le courant de produit gazeux s'élevant dans cette colonne arrivant à contre-courant du condensat formé à l'opération de traitement suivante des produits de dissociation du dichloréthane et étant renvoyé à la partie supérieure de la colonne de trempe, puis lavé et refroidi, procédé caractérisé en ce que :
b) le produit de a) est obtenu en buées de la colonne de trempe à une pression absolue de 9-12 bar et a une température de 120-140°C, et
c) ces buées sont portées par un refroidissement indirect au moins jusqu'à leur point de rosée,
et on utilise dans des échangeurs de chaleur fonctionnant en parallèle, comme milieux d'échange de chaleur ;
c₁) 20 à 40 % en volumes des buées avec le 1,2-chloréthane amené au four de dissociation,
c₂) 10 à 20 % en volumes des buées avec l'air servant à la combustion pour enflammer le four, et
c₃) 30 à 50 % en volumes des buées avec le produit de fond de la colonne prévue pour la séparation par distillation du chlorure d'hydrogène, et le cas échéant
c₄) une partie restante des buées avec le chlorure d'hydrogène liquide.
